# EUROPEAN PATENT APPLICATION

(11) **EP 1 614 682 A1**
(43) Date of publication of application: **11.01.2006**
(21) Application number: 04716036.1
(22) Date of filing: 01.03.2004
(51) Int. Cl.: C07D 215/14

(54) **PROCESS FOR PRODUCING QUINOLINE COMPOUND**

(30) Priority: 04.04.2003 JP 2003102134
(71) Applicant: Nissan Chemical Industries, Ltd., Chiyoda-ku, Tokyo 101-0054 (JP)
(72) Inventor: FUKUMOTO, Takashi, Kuraray Co., Ltd., Kitakanbara-gun, Niigata 959-2691 (JP); NAGASHIMA, Kensuke, Kuraray Co., Ltd., Kurashiki-shi, Okayama 710-8622 (JP)
(74) Representative: von Kreisler, Alek
(86) International application number: PCT/JP2004/002464
(87) International publication number: WO 2004/089907

(57) **Abstract**

By a production method of a quinoline compound represented by the formula (I), which comprises reacting quinolinecarbaldehyde represented by the formula (II) with an imine compound represented by the formula (III) and then subjecting the resulting compound to hydrolysis, a quinoline compound useful as a synthetic intermediate for pharmaceutical agents, agricultural chemicals and the like can be efficiently and industrially advantageously produced in a short step using a starting material industrially easily available and easy to handle: 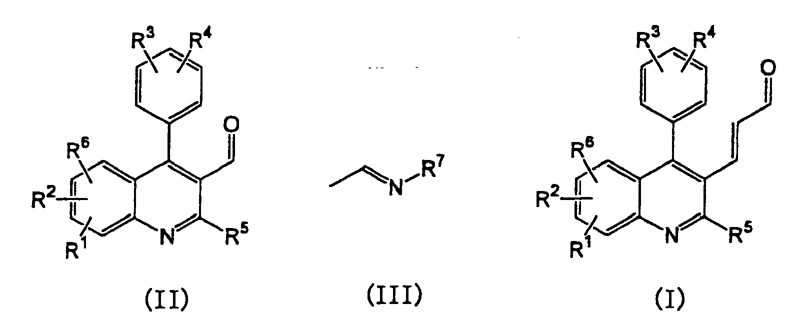
wherein R¹ , R², R³, R⁴, R⁵, R⁶ and R⁷ are as defined in the description.

## Description

### Technical Field

The present invention relates to a production method of a quinoline compound represented by the formula (I) to be mentioned below. The quinoline compound obtained in the present invention is useful as a synthetic intermediate for pharmaceutical agents, agricultural chemicals and the like. For example, (E)-3-(4'-(4"-fluorophenyl)-2'-cyclopropylquinolin-3'-yl)propenaldehyde is a synthetic intermediate for a quinoline compound known as an inhibitor of HMG-CoA reductase, which is a rate-limiting enzyme of cholesterol biosynthesis.

### Background Art

Conventionally, as production methods of quinoline compounds, for example, (E)-3-(4'-(4"-fluorophenyl)-2'-cyclopropylquinolin-3'-yl)propenaldehyde, (1) a method comprising reacting cis-1-ethoxy-2-(tri-n-butylstannyl)ethylene with butyl lithium in tetrahydrofuran, then reacting the reactant with 4-(4'-fluorophenyl)-2-cyclopropylquinoline-3-carbaldehyde at - 60°C to -78°C and hydrolyzing the obtained vinyl ether form in the presence of an acid catalyst (see JP-A-1-279866), (2) a method comprising reacting 4-(4'-fluorophenyl)-2-cyclopropylquinoline-3-carbaldehyde with alkoxycarbonylmethyl phosphonate to give the corresponding α,β-unsaturated carboxylate, then reducing an ester moiety of this compound using, for example, a metal hydride such as diisobutylaluminum hydride and the like to convert to an alcohol, and oxidizing the alcohol using an oxidizing agent such as activated manganese dioxide and the like (see JP-A-1-279866), and (3) a method comprising reacting 4-(4'-fluorophenyl)-2-cyclopropylquinoline-3-carbaldehyde with (1,3-dioxolan-2-ylmethyl)triphenylphosphonium bromide or diethylphosphonoacetaldehyde diethyl acetal in the presence of tert-butyl lithium to give the corresponding α,β-unsaturated aldehyde acetal, and then hydrolyzing an acetal moiety of the compound (see JP-A-2001-316369) are known.

With regard to the above-mentioned method (1), however, the organic tin compound to be used is industrially difficult to obtain, and the reaction needs to be carried out at an extremely low temperature of - 60°C to -78°C, thus requiring special reaction equipment. As for the above-mentioned method (2), metal hydride to be used for reducing the ester moiety is difficult to handle. In addition, in the above-mentioned method (2) and method (3), a number of steps are necessary before obtaining the object products. Therefore, these methods are hardly said to be industrially advantageous production methods of quinoline compounds such as (E)-3-(4'-(4"-fluorophenyl)-2'-cyclopropylquinolin-3'-yl)propenaldehyde and the like.

Therefore, it is an object of the present invention to provide a method capable of efficiently and industrially advantageously manufacturing a quinoline compound in a short step using a starting material industrially easily available and easy to handle.

### Disclosure of the Invention

According to the present invention, the above-mentioned object can be achieved by providing a production method of a quinoline compound represented by the formula (I) 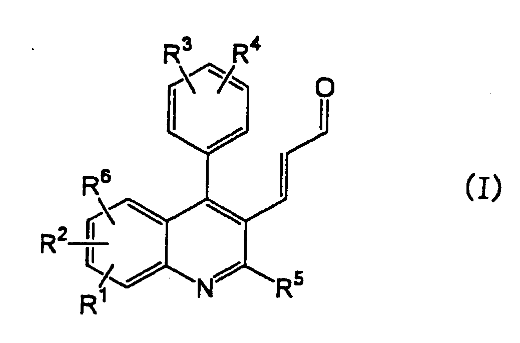
wherein R¹, R² , R³, R⁴, R⁵ and R⁶ are each a hydrogen atom, a halogen atom, an optionally protected hydroxyl group, an alkyl group optionally having substituent(s), an aryl group optionally having substituent(s), an aralkyl group optionally having substituent(s), an alkoxyl group optionally having substituent(s) or an aryloxy group optionally having substituent(s) [in this description, to be sometimes to be abbreviated as quinoline compound (I)],
which comprises reacting a quinolinecarbaldehyde represented by the formula (II) 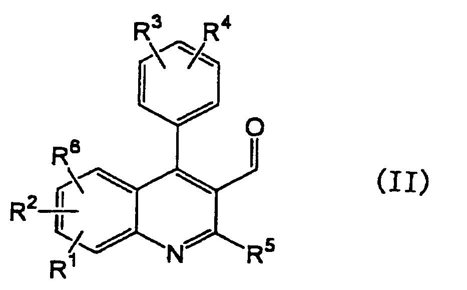
wherein R¹, R², R³, R⁴, R⁵ and R⁶ are as defined above [hereinafter to be referred to as quinolinecarbaldehyde (II)]
with an imine compound represented by the formula (III) 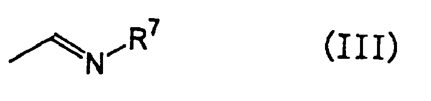
wherein R⁷ is an alkyl group optionally having substituent(s) [hereinafter to be referred to as imine compound (III)], and then hydrolyzing the resulting compound.

In a preferable embodiment, the above-mentioned R¹, R², R³ and R⁶ are each a hydrogen atom, R⁴ is a halogen atom, and R⁵ is an alkyl group having 1 to 6 carbon atoms.

In a more preferable embodiment, the above-mentioned R¹, R², R³ and R⁶ are each a hydrogen atom, R⁴ is a fluorine atom, and R⁵ is an alkyl group having 1 to 6 carbon atoms.

In a still more preferable embodiment, the above-mentioned R¹, R², R³ and R⁶ are each a hydrogen atom, R⁴ is a fluorine atom, and R⁵ is an isopropyl group or a cyclopropyl group.

### Best Mode for Embodying the Invention

In this description, as the halogen atom represented by each of R¹, R², R³, R⁴, R⁵ and R⁶, for example, fluorine atom, chlorine atom, bromine atom, iodine atom and the like can be mentioned, with preference given to fluorine atom.

The hydroxyl group represented by each of R¹, R², R³, R⁴, R⁵ and R⁶ may be protected, and the hydroxyl-protecting group is not particularly limited as long as it is generally used for protecting hydroxyl group. For example, aralkyl groups such as benzyl group and the like; tri-substituted silyl groups such as trimethylsilyl group, tert-butyldimethylsilyl group, tert-butyldiphenylsilyl group and the like; ether protecting groups such as methoxymethyl group, 1-ethoxyethyl group, tetrahydrofuranyl group, tetrahydropyranyl group and the like, and the like can be mentioned.

The alkyl group represented by each of R¹, R², R³, R⁴, R⁵, R⁶ and R⁷ may be linear, branched chain or cyclic. Preferably, it is a linear, branched chain or cyclic alkyl group having 1 to 6 carbon atoms, such as methyl group, ethyl group, n-propyl group, isopropyl group, n-butyl group, isobutyl group, tert-butyl group, n-pentyl group, n-hexyl group, cyclopropyl group, cyclohexyl group and the like can be mentioned. These alkyl groups optionally have substituent(s), and as the substituent, for example, hydroxyl group; alkoxyl groups preferably having 1 to 4 carbon atoms, such as methoxy group, ethoxy group, propoxy group, butoxy group and the like; aryl groups preferably having 6 to 10 carbon atoms, which optionally has substituent(s), such as phenyl group, p-methoxyphenyl group, p-chlorophenyl group and the like; halogen atoms such as fluorine atom, chlorine atom, bromine atom, iodine atom and the like, and the like can be mentioned.

The aryl group represented by each of R¹, R², R³, R⁴, R⁵ and R⁶ is preferably an aryl group having 6 to 10 carbon atoms, for example, phenyl group, naphthyl group or the like.

As the aralkyl group represented by each of R¹, R², R³, R⁴, R⁵ and R⁶, an aralkyl group wherein the alkyl moiety is an alkyl group preferably having 1 to 6 carbon atoms and the aryl moiety is an aryl group preferably having 6 to 10 carbon atoms can be mentioned, which is, for example, benzyl group, naphthylmethyl group or the like. These aryl group and aralkyl group may have a substituent(s), and as the substituent, for example, hydroxyl group; alkyl groups preferably having 1 to 6 carbon atoms, such as methyl group, ethyl group, n-propyl group, isopropyl group, n-butyl group, isobutyl group, tert-butyl group, n-pentyl group, n-hexyl group and the like; alkoxyl groups preferably having 1 to 4 carbon atoms, such as methoxy group, ethoxy group, propoxy group, butoxy group and the like; aryl groups preferably having 6 to 10 carbon atoms, which optionally has substituent(s), such as phenyl group, p-methoxyphenyl group, p-chlorophenyl group and the like; halogen atoms such as a fluorine atom, chlorine atom, bromine atom, iodine atom and the like, and the like can be mentioned.

As the alkoxyl group represented by each of R¹, R², R³, R⁴, R⁵ and R⁶, a linear or branched chain alkoxyl group preferably having 1 to 4 carbon atoms can be mentioned. For example, methoxy group, ethoxy group, propoxy group, butoxy group and the like can be mentioned. These alkoxyl groups may have substituent(s), and as the substituent, for example, hydroxyl group; alkoxyl groups preferably having 1 to 4 carbon atoms, such as methoxy group, ethoxy group, propoxy group, butoxy group and the like; aryl groups preferably having 6 to 10 carbon atoms, which optionally has substituent(s), such as phenyl group, p-methoxyphenyl group, p-chlorophenyl group and the like, and the like can be mentioned.

As the aryloxy group represented by each of R¹, R², R³, R⁴, R⁵ and R⁶, an aryloxy group wherein the aryl moiety is an aryl group preferably having 6 to 10 carbon atoms, such as phenoxy group, naphthyloxy group and the like can be mentioned. These aryloxy groups may have substituent(s), and as the substituent, for example, hydroxyl group; alkyl groups preferably having 1 to 6 carbon atoms, such as methyl group, ethyl group, n-propyl group, isopropyl group, n-butyl group, isobutyl group, tert-butyl group and the like; alkoxyl groups preferably having 1 to 4 carbon atoms, such as methoxy group, ethoxy group, propoxy group, butoxy group and the like; and aryl groups preferably having 6 to 10 carbon atoms, which optionally has substituent(s), such as phenyl group, p-methoxyphenyl group, p-chlorophenyl group and the like can be mentioned.

As the quinolinecarbaldehyde (II) and quinoline compound (I), compounds wherein R¹ , R², R³ and R⁶ are each a hydrogen atom, R⁴ is a halogen atom, and R⁵ is an alkyl group having 1 to 6 carbon atoms are preferable.

As the quinolinecarbaldehyde (II) and quinoline compound (I), compounds wherein R¹, R² , R³ and R⁶ are each a hydrogen atom, R⁴ is a fluorine atom, and R⁵ is an alkyl group having 1 to 6 carbon atoms are more preferable.

As the quinolinecarbaldehyde (II) and quinoline compound (I), compounds wherein R¹, R², R³ and R⁶ are each a hydrogen atom, R⁴ is a fluorine atom, and R⁵ is an isopropyl group or a cyclopropyl group are still more preferable.

As the imine compound (III), a compound wherein R⁷ is an ethyl group, a n-propyl group, an isopropyl group, a n-butyl group, an isobutyl group, a tert-butyl group, a cyclopentyl group or a cyclohexyl group is preferable.

As the imine compound (III), a compound wherein R⁷ is an isopropyl group, a n-butyl group, an isobutyl group, a tert-butyl group or a cyclohexyl group is more preferable.

The production method of quinoline compound (I) of the present invention comprises reacting quinolinecarbaldehyde (II) with imine compound (III), which is then followed by hydrolysis.

According to the method of the present invention, quinoline compound (I) can be efficiently and industrially advantageously produced in a short step using a starting material industrially easily available and easy to handle.

First, a step for reacting quinolinecarbaldehyde (II) with imine compound (III) [hereinafter to be referred to as Step 1] is explained.

In Step 1, the presence of a base is preferable, and as the base, for example, alkali metal carbonates such as sodium carbonate, potassium carbonate and the like; alkali metal hydroxides such as sodium hydroxide, potassium hydroxide and the like; alkali metal hydrides such as lithium hydride, sodium hydride, potassium hydride and the like; organic lithium compounds such as methyllithium, ethyllithium, n-butyllithium, sec-butyllithium, tert-butyllithium, phenyllithium and the like; alkylmagnesium halides such as methylmagnesium chloride, ethylmagnesium bromide and the like; metal amides such as lithium amide, sodium amide, potassium amide, lithium diethylamide, lithium di(isopropyl)amide, lithium bis(trimethylsilyl)amide, sodium bis(trimethylsilyl)amide, potassium bis(trimethylsilyl)amide, bromomagnesium di(isopropyl)amide and the like; metal alkoxides such as lithium methoxide, sodium methoxide, sodium ethoxide, sodium tert-butoxide, potassium tert-butoxide and the like, and the like can be mentioned. Of these, sodium hydride, n-butyl lithium and lithium di(isopropyl)amide are preferably used from the aspects of availability, easy handlability, reactivity and the like. The amount of the base to be used is preferably 0.1- to 10-fold mol relative to quinolinecarbaldehyde (II). More preferably, the amount is 0.1- to 5-fold mol relative to quinolinecarbaldehyde (II) from the aspects of easy work up, economical efficiency and the like.

As the imine compound (III) to be used in Step 1, for example, ethylideneisopropylamine, ethylidene n-butylamine, ethylideneisobutylamine, ethylidene tert-butylamine, ethylidenecyclohexylamine and the like can be mentioned. The amount of the imine compound (III) to be used is preferably 0.1- to 10-fold mol relative to quinolinecarbaldehyde (II). More preferably, the amount is 0.1- to 5-fold mol relative to quinolinecarbaldehyde (II) from the aspects of easy work up, economical efficiency and the like.

Step 1 is preferably performed in the presence of a solvent. The solvent is free of particularly limitation as long as it does not adversely affect the reaction. For example, aliphatic hydrocarbons such as pentane, hexane, heptane, cyclohexane, methylcyclohexane and the like; aromatic hydrocarbons such as benzene, toluene, xylene and the like; alcohols such as methanol, ethanol, isopropanol, n-butanol, tert-butanol and the like; ethers such as diethyl ether, diisopropyl ether, tert-butyl methyl ether, tetrahydrofuran, dioxane and the like; nitriles such as benzonitrile and the like; nitrogen-containing aromatic compounds such as pyridine and the like; amides such as dimethylformamide, N-methylpyrrolidone, N,N'-dimethylimidazolidinone, hexamethylphosphoric triamide and the like, and the like can be mentioned. These solvents may be used alone or in a mixture of two or more kinds thereof. Of these, diisopropyl ether, tert-butyl methyl ether and tetrahydrofuran are preferable from the aspects of easy handlability, availability, reactivity and the like. The amount of the solvent to be used is preferably within the range of 1- to 50-fold by mass relative to quinolinecarbaldehyde (II). More preferably, the amount is within the range of 1- to 10-fold by mass from the aspects of economical efficiency and the like.

While the reaction temperature of Step 1 varies depending on the kind of quinolinecarbaldehyde (II) to be used, the kind of imine compound (III) to be used, the kind of the base to be used, the kind of the solvent to be used and the like, it is generally preferably within the range of -100°C to 130°C, more preferably - 30°C to 100°C.

While the reaction time of Step 1 varies depending on the reaction temperature and the like, it is generally preferably within the range of 0.1-24 hrs. The reaction is preferably carried out under an inactive gas atmosphere of nitrogen, helium, argon or the like.

The operation method of Step 1 is not particularly limited. For example, a method comprising adding a base to a mixture of quinolinecarbaldehyde (II), imine compound (III) and a solvent, a method comprising adding a mixture of quinolinecarbaldehyde (II), imine compound (III) and a solvent to a base, a method comprising adding imine compound (III) to a mixture of quinolinecarbaldehyde (II), a base and a solvent, a method comprising simultaneously adding imine compound (III) and a base separately to a mixture of quinolinecarbaldehyde (II) and a solvent, a method comprising adding a mixture of imine compound (III) and a base to a mixture of quinolinecarbaldehyde (II) and a solvent and the like, each of which is performed under an inactive gas atmosphere of nitrogen, helium, argon or the like, can be mentioned. The reaction of Step 1 can be quenched by adding water to the reaction system, and the like. The reaction of Step 1 can be carried out by a batch method or a continuous method.

Imine compound (III), for example, ethylidene tert-butylamine, to be used in Step 1 can be easily produced by reacting tert-butylamine and acetaldehyde (see US Patent No. 2582128).

The reaction mixture obtained by Step 1 can be isolated and purified by a method generally used for isolation and purification of organic compounds. In the present invention, however, the reaction mixture is subjected as it is to the next step described in the following.

Next, a step for obtaining quinoline compound (I) by hydrolyzing the reaction mixture obtained in Step 1 [hereinafter to be referred to as Step 2] is explained.

For Step 2, general hydrolysis conditions for reacting, for example, an acid, in a solvent containing water can be employed. While the amount of water to be used is not particularly limited, it is generally preferably not less than 1-fold mol, more preferably within the range of 1- to 1000-fold mol, relative to quinolinecarbaldehyde (II) used in Step 1.

When an acid is used in Step 2, as the acid, for example, mineral acids such as hydrochloric acid, sulfuric acid, phosphoric acid and the like; organic acids such as acetic acid, propionic acid, oxalic acid, methanesulfonic acid, toluenesulfonic acid and the like, a hydrate thereof or a salt thereof, and the like can be mentioned. While the amount of the acid to be used is not particularly limited, it is generally preferably within the range of 0.01- to 5-fold mol relative to the amount of quinolinecarbaldehyde (II) used as a starting material in Step 1.

As the solvent to be used in Step 2, the same solvent as used in Step 1 can be used. The amount of the solvent to be used is preferably within the range of 1-to 50-fold by mass relative to the amount of quinolinecarbaldehyde (II) used as a starting material in Step 1. More preferably, the amount is within the range of 1- to 10-fold by mass relative to the amount of quinolinecarbaldehyde (II), from the aspects of economic efficiency and the like.

While the reaction temperature of Step 2 varies depending on the kind of the acid to be used, the kind of the solvent to be used and the like, it is generally preferably within the range of 0°C to 100°C. While the reaction time varies depending on the reaction temperature, it is generally preferably within the range of 1-24 hrs.

The operation method of Step 2 is not particularly limited. For example, a method comprising mixing the reaction mixture obtained in Step 1, water, an acid and a solvent, and stirring the mixture at a predetermined temperature can be mentioned. The reaction of Step 2 can be carried out by a batch method or a continuous method.

The quinoline compound (I) obtained in this way can be isolated and purified by a method generally used for isolation and purification of organic compounds. For example, a base such as aqueous sodium hydrogencarbonate solution, sodium methoxide and the like is added as necessary to a reaction mixture after completion of the reaction to neutralize the acid, the reaction mixture is extracted with an organic solvent such as diethyl ether, ethyl acetate, methylene chloride and the like, and the organic layer is concentrated to give a crude product, which can be then used as it is as a synthetic intermediate for a pharmaceutical product such as quinoline compound and the like. Where necessary, the crude product can be further purified by recrystallization, column chromatography and the like.

### Examples

The present invention is explained in detail in the following by referring to Examples, which are not to be construed as limitative.

### Reference Example 1

tert-Butylamine (166.0 g, 2.27 mol) was charged in a 500 ml three-neck flask equipped with a thermometer, a dropping funnel and a magnetic stirrer and cooled to 2°C. Acetaldehyde (100.0 g, 2.27 mol) was added dropwise from the dropping funnel over 2 hrs with stirring. After completion of the dropwise addition, the mixture was further stirred for 2 hrs, and potassium hydroxide (30.0 g, 0.53 mol) was added. The mixture was stirred for 30 min and left standing to allow separation of the organic layer from the aqueous layer. Potassium hydroxide (10.0 g, 0.18 mol) was added to the obtained organic layer, and the mixture was stood in a refrigerator for 15 hrs. The upper layer was separated and distilled under atmospheric pressure (boiling point: 77-82°C) to give ethylidene tert-butylamine (182.3 g, 1.84 mol, yield 81.0%).

### Reference Example 2

Cyclohexylamine (24.8 g, 250 mmol) was charged in a 200 ml three-neck flask equipped with a thermometer, a dropping funnel and a magnetic stirrer and cooled to 2°C. Acetaldehyde (11.0 g, 250 mmol) was added dropwise from the dropping funnel over 2 hrs with stirring. After completion of the dropwise addition, the mixture was further stirred for 2 hrs, and toluene (50 ml) was added. The mixture was stirred for 30 min and left standing to allow separation of the organic layer from the aqueous layer. The obtained organic layer was distilled under reduced pressure (boiling point: 47-48°C/1.6 kPa) to give ethylidenecyclohexylamine (125.8 g, 206 mmol, yield 82.5%).

### Reference Example 3

n-Butylamine (18.3 g, 250 mmol) was charged in a 200 ml three-neck flask equipped with a thermometer, a dropping funnel and a magnetic stirrer and cooled to 2°C. Acetaldehyde (11.0 g, 250 mmol) was added dropwise from the dropping funnel over 2 hrs with stirring. After completion of the dropwise addition, the mixture was further stirred for 2 hrs, and diisopropyl ether (50 ml) was added. The mixture was stirred for 30 min and left standing to allow separation of the organic layer from the aqueous layer. The obtained organic layer was distilled under atmospheric pressure (boiling point: 98-105°C) to give ethylidene n-butylamine (20.0 g, 202 mmol, yield 80.7%).

### Example 1

4-(4'-Fluorophenyl)-2-cyclopropylquinoline-3-carbaldehyde (2.0 g, 6.9mmol), ethylidene tert-butylamine (1.4 g, 14.1 mmol) and tetrahydrofuran (10 ml) were charged in a 50 ml three-neck flask equipped with a thermometer, a magnetic stirrer and a nitrogen balloon and the mixture was heated to 63°C. Sodium hydride (60 mass %, 0.44 g, 11.0 mmol) was added over 3 hrs. After completion of the addition, the mixture was further stirred for 8 hrs. A part of the obtained reaction mixture was poured into the 10 mass % aqueous acetic acid solution, and an analysis by internal standard methods was performed using high performance liquid chromatography. As a result, (E)-3-(4'-(4"-fluorophenyl)-2'-cyclopropylquinolin-3'-yl)propenaldehyde (1.5 g, 4.7 mmol, yield 68.0%) was found to have been produced.
melting point: 124-132°C
¹H-NMR (600 MHz, CDCl₃, TMS, ppm) .δ: 1.08-1.13(2H,m), 1.41-1.45(2H,m), 2.32-2.38(1H,m), 6.45(1H,dd,J=8,16Hz), 7.22-7.25 (4H,m), 7.35-7.39 (2H,m), 7.56(1H,d,J=16Hz), 7.67(1H,ddd,J=3,6,8Hz), 7.98(1H,d,J=8Hz), 9.51(1H,d,J=8Hz)

### Example 2

The same operation as in Example 1 was performed except that ethylidenecyclohexylamine (1.7 g, 13.8 mmol) was used instead of ethylidene tert-butylamine (1.4 g, 14.1 mmol). A part of the obtained reaction mixture was poured into the 10 mass % aqueous acetic acid solution, and an analysis by internal standard methods was performed using high performance liquid chromatography. As a result, (E) -3- (4'-(4"-fluorophenyl)-2'-cyclopropylquinolin-3'-yl)propenaldehyde (1.7 g, 5.4 mmol, yield 78.3%) was found to have been produced.

### Example 3

The same operation as in Example 1 was performed except that ethylidene n-butylamine (1.4 g, 14.1 mmol) was used instead of ethylidene tert-butylamine (1.4 g, 14.1 mmol). A part of the obtained reaction mixture was poured into the 10 mass % aqueous acetic acid solution, and an analysis by internal standard methods was performed using high performance liquid chromatography. As a result, (E)-3-(4'-(4"-fluorophenyl)-2'-cyclopropylquinolin-3'-yl)propenaldehyde (1.3 g, 4.2 mmol, yield 60.3%) was found to have been produced.

### Industrial Applicability

According to the present invention, a quinoline compound useful as a synthetic intermediate for a pharmaceutical agent, an agricultural chemical and the like, for example, (E)-3-(4'-(4"-fluorophenyl)-2'-cyclopropylquinolin-3'-yl)propenaldehyde, an important synthetic intermediate for a quinoline compound known as an inhibitor of HMG-CoA reductase, which is a rate-limiting enzyme of cholesterol biosynthesis, can be produced efficiently and industrially advantageously in a short step.

This application is based on patent application No. 2003-102134 filed in Japan, the contents of which are hereby incorporated by reference.

## Claims

1. A production method of a quinoline compound represented by the formula (I) 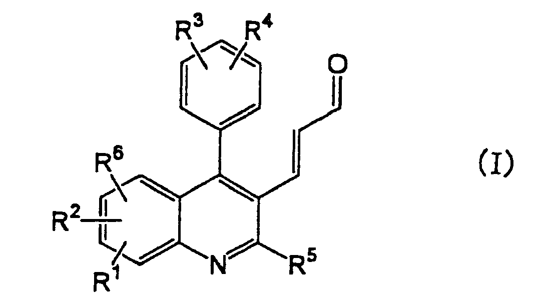
wherein R¹, R², R³, R⁴, R⁵ and R⁶ are each a hydrogen atom, a halogen atom, an optionally protected hydroxyl group, an alkyl group optionally having substituent(s), an aryl group optionally having substituent(s), an aralkyl group optionally having substituent(s), an alkoxyl group optionally having substituent(s) or an aryloxy group optionally having substituent(s),
which comprises reacting a quinolinecarbaldehyde represented by the formula (II) 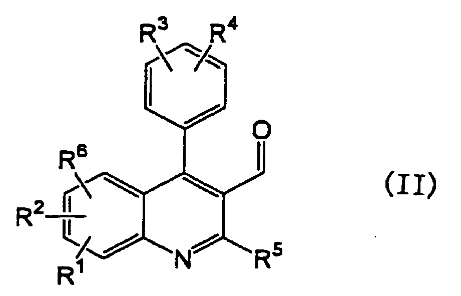
wherein R¹, R², R³, R⁴, R⁵ and R⁶ are as defined above, with an imine compound represented by the formula (III) 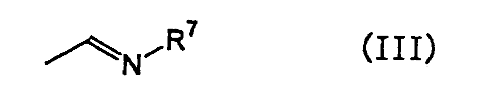
wherein R⁷ is an alkyl group optionally having substituent(s), and then hydrolyzing the resulting compound.
